# EUROPEAN PATENT APPLICATION

(11) **EP 3 925 617 A1**
(43) Date of publication of application: **22.12.2021**
(21) Application number: 20739841.3
(22) Date of filing: 29.06.2020
(51) Int. Cl.: A61K 39/00, A61P 15/00, A61P 5/24, C07K 14/28, C07K 7/06, C07K 14/705, C12N 15/63

(54) **RECOMBINANT PROTEIN FOR ELIMINATING BOAR TAINT AND VACCINE COMPOSITION COMPRISING SAME**

(30) Priority: 24.03.2020 KR 20200035897
(71) Applicant: Bioapplications Inc., Pohang-si, Gyeongsangbuk-do 37668 (KR)
(72) Inventor: SOHN, Eun-Ju, Pohang-Si, Gyeongsangbuk-do 37668 (KR); LEE, Yong Jik, Pohang-Si, Gyeongsangbuk-do 37668 (KR); LEE, Sangmin, Pohang-Si, Gyeongsangbuk-do 37558 (KR)
(74) Representative: Dehns
(86) International application number: PCT/KR2020/008420
(87) International publication number: WO 2021/194013

(57) **Abstract**

The present invention relates to a vaccine composition for removing boar taint, the vaccine composition including a recombinant protein in which a cholera toxin B subunit (CTB) and n gonadotropin-releasing hormones (GnRH) are fused, and more specifically, provides a recombinant protein for removing boar taint to induce antibodies against GnRH, a recombinant vector for producing the same, a vaccine composition for removing boar taint, the vaccine composition including the recombinant protein, and a method for removing boar taint using the same. The vaccine composition according to the present invention induces antibodies against GnRH in an individual and has the effect of atrophying the testes through the induction of antibodies. Therefore, the present invention can be usefully used to remove boar taint by immunologically castrating a boar at low cost and with high safety and minimal side effects.

<110>    BioApplications Inc.

<120>    Recombinant Protein for Removing Boar Taint and Vaccine
         Composition Comprising the Same

<130>    MPCT20-062

<150>    KR 10-2020-0035897
<151>    2020-03-24

<160>    13

<170>    KoPatentIn 3.0

<210>    1
<211>    10
<212>    PRT
<213>    Artificial Sequence

<220>
<223>    Amino acid sequence of GnRH


<400>    1
Glu His Trp Ser Tyr Gly Leu Arg Pro Gly
  1               5                  10


<210>    2
<211>    78
<212>    PRT
<213>    Artificial Sequence

<220>
<223>    Amino acid sequence of 6X GnRH


<400>    2
Ala Gly Ala Glu His Trp Ser Tyr Gly Leu Arg Pro Gly Ala Gly Ala
  1               5                  10                  15

Glu His Trp Ser Tyr Gly Leu Arg Pro Gly Ala Gly Ala Glu His Trp
             20                  25                  30

Ser Tyr Gly Leu Arg Pro Gly Ala Gly Ala Glu His Trp Ser Tyr Gly
         35                  40                  45

Leu Arg Pro Gly Ala Gly Ala Glu His Trp Ser Tyr Gly Leu Arg Pro
     50                  55                  60

Gly Ala Gly Ala Glu His Trp Ser Tyr Gly Leu Arg Pro Gly
 65                  70                  75


<210>    3
<211>    234
<212>    DNA
<213>    Artificial Sequence

<220>
<223>    DNA sequence of 6X GnRH

<400>    3
gcaggagcag aacattggtc ttacggtctt agacctggag ctggagctga acattggagt        60

tatggtttaa gacctggagc tggagctgaa cattggtctt atggacttag accaggtgca       120

ggtgctgagc attggtcata tggattgaga ccaggagctg gtgctgagca ttggtcttat       180

ggtctaagac ctggagctgg agctgaacat tggtcttacg gactcagacc cggt             234


<210>    4
<211>    102
<212>    PRT
<213>    Artificial Sequence

<220>
<223>    Amino acid sequence of CTB


<400>    4
Thr Pro Gln Asn Ile Thr Asp Leu Cys Ala Glu Tyr His Asn Thr Gln
1               5                   10                  15

Ile His Thr Leu Asn Asp Lys Ile Phe Ser Tyr Thr Glu Ser Leu Ala
            20                  25                  30

Gly Lys Arg Glu Met Ala Ile Ile Thr Phe Lys Asn Gly Ala Thr Phe
        35                  40                  45

Gln Val Glu Val Pro Gly Ser Gln His Ile Asp Ser Gln Lys Lys Ala
    50                  55                  60

Ile Glu Arg Met Lys Asp Thr Leu Arg Ile Ala Tyr Leu Thr Glu Ala
65                  70                  75                  80

Lys Val Glu Lys Leu Cys Val Trp Asn Asn Lys Thr Pro His Ala Ile
                85                  90                  95

Ala Ala Ile Ser Met Ala
            100


<210>    5
<211>    306
<212>    DNA
<213>    Artificial Sequence

<220>
<223>    DNA sequence of CTB


<400>    5
actcctcaga acataaccga tttgtgcgct gaataccata acactcaaat acacacactc        60

aacgataaga ttttctctta cactgaatca cttgctggaa agagagagat ggcaattatc       120

acctttaaaa acggagctac tttccaggtt gaagtgcctg gtagtcaaca tatcgattca       180

cagaagaaag caatagagag gatgaaggat acacttagga ttgcttattt gaccgaagca       240

aaagttgaga aactctgtgt gtggaataac aaaacccctc acgctatcgc tgctatctct       300

atggcc                                                                  306

<210>    6
<211>    229
<212>    PRT
<213>    Artificial Sequence

<220>
<223>    Amino acid sequence of pFc2


<400>    6
Arg Ala Val Gly Arg Pro Cys Pro Ile Cys Pro Ala Cys Glu Gly Pro
1               5                   10                  15

Gly Pro Ser Ala Phe Ile Phe Pro Pro Lys Pro Lys Asp Thr Leu Met
            20                  25                  30

Ile Ser Arg Thr Pro Gln Val Thr Cys Val Val Val Asp Val Ser Gln
        35                  40                  45

Glu Asn Pro Glu Val Gln Phe Ser Trp Tyr Val Asp Gly Val Glu Val
    50                  55                  60

His Thr Ala Gln Thr Arg Pro Lys Glu Ala Gln Phe Asn Ser Thr Tyr
65                  70                  75                  80

Arg Val Val Ser Val Leu Pro Ile Gln His Glu Asp Trp Leu Lys Gly
                85                  90                  95

Lys Glu Phe Glu Cys Lys Val Asn Asn Lys Asp Leu Pro Ala Pro Ile
            100                 105                 110

Thr Arg Ile Ile Ser Lys Ala Lys Gly Pro Ser Arg Glu Pro Gln Val
        115                 120                 125

Tyr Thr Leu Ser Pro Ser Ala Glu Glu Leu Ser Arg Ser Lys Val Ser
    130                 135                 140

Ile Thr Cys Leu Val Thr Gly Phe Tyr Pro Pro Asp Ile Asp Val Glu
145                 150                 155                 160

Trp Lys Ser Asn Gly Gln Pro Glu Pro Glu Gly Asn Tyr Arg Thr Thr
                165                 170                 175

Pro Pro Gln Gln Asp Val Asp Gly Thr Tyr Phe Leu Tyr Ser Lys Leu
            180                 185                 190

Ala Val Asp Lys Ala Ser Trp Gln Arg Gly Asp Pro Phe Gln Cys Ala
        195                 200                 205

Val Met His Glu Ala Leu His Asn His Tyr Thr Gln Lys Ser Val Ser
    210                 215                 220

Lys Thr Gln Gly Lys
225


<210>    7
<211>    681
<212>    DNA
<213>    Artificial Sequence

<220>
<223>    DNA sequence of pFc2

<400>    7
   gttggaagac catgccctat atgtcctgct tgtgaaggtc caggtccctc tgcttttata      60
   ttcccaccaa agccgaagga taccttgatg atttcacgta caccacaagt tacttgtgtt     120
   gttgtggatg tttcacaaga aaatcctgag gtacaattca gctggtatgt tgatggggta     180
   gaagtgcaca ctgcacagac tcgaccaaag gaggcccagt ttaactcgac ttatagagtt     240
   gtttctgttc tcccaatcca acacgaagat tggctgaagg gcaaggaatt tgaatgcaag     300
   gttaacaata aagatctacc agcaccaatt accaggatta tttctaaggc aaaaggaccc     360
   tccagagagc cccaagttta cacattgtct ccttctgctg aggagcttag tagaagtaaa     420
   gtgagcatta cctgcttagt gacgggattc taccctccag acatcgacgt cgaatggaaa     480
   tctaatggtc aacctgagcc agaaggtaac tataggacta ctccaccaca acaggacgtc     540
   gatggcacat actttcttta ttcaaaactt gctgtcgata aggcaagttg gcaaagagga     600
   gatccatttc agtgtgctgt aatgcatgag gctttgcata atcattatac acagaaatca     660
   gtttctaaaa cacaaggaaa a                                                681

<210>    8
<211>    7
<212>    PRT
<213>    Artificial Sequence

<220>
<223>    Amino acid sequence of BiP

<400>    8
Ile Glu Glu Ala Thr Lys Leu
 1               5

<210>    9
<211>    273
<212>    DNA
<213>    Artificial Sequence

<220>
<223>    DNA sequence of BiP

<400>    9
atggctcgct cgtttggagc taacagtacc gttgtgttgg cgatcatctt cttcggtgag      60

tgattttccg atcttcttct ccgatttaga tctcctctac attgttgctt aatctcagaa     120

ccttttttcg ttgttcctgg atctgaatgt gtttgtttgc aatttcacga tcttaaaagg     180

ttagatctcg attggtattg acgattggaa tctttacgat ttcaggatgt ttatttgcgt     240

tgtcctctgc aatagaagag gctacgaagt taa                                  273

<210>    10
<211>    4
<212>    PRT
<213>    Artificial Sequence

<220>
<223>    Amino acid sequence of HDEL


<400>    10
His Asp Glu Leu
1


<210>    11
<211>    12
<212>    DNA
<213>    Artificial Sequence

<220>
<223>    DNA sequence of HDEL


<400>    11
cacgatgagc tc                                                          12


<210>    12
<211>    428
<212>    PRT
<213>    Artificial Sequence

<220>
<223>    Full amino acid sequence of 1300-BiP:CTB:GnRH:pFc2:HDEL


<400>    12
Ile Glu Glu Ala Thr Lys Leu Arg Ile Leu Thr Pro Gln Asn Ile Thr
1               5                   10                  15

Asp Leu Cys Ala Glu Tyr His Asn Thr Gln Ile His Thr Leu Asn Asp
            20                  25                  30

Lys Ile Phe Ser Tyr Thr Glu Ser Leu Ala Gly Lys Arg Glu Met Ala
        35                  40                  45

Ile Ile Thr Phe Lys Asn Gly Ala Thr Phe Gln Val Glu Val Pro Gly
    50                  55                  60

Ser Gln His Ile Asp Ser Gln Lys Lys Ala Ile Glu Arg Met Lys Asp
65                  70                  75                  80

Thr Leu Arg Ile Ala Tyr Leu Thr Glu Ala Lys Val Glu Lys Leu Cys
                85                  90                  95

Val Trp Asn Asn Lys Thr Pro His Ala Ile Ala Ala Ile Ser Met Ala
            100                 105                 110

Thr Ser Ala Gly Ala Glu His Trp Ser Tyr Gly Leu Arg Pro Gly Ala
        115                 120                 125

Gly Ala Glu His Trp Ser Tyr Gly Leu Arg Pro Gly Ala Gly Ala Glu
    130                 135                 140

His Trp Ser Tyr Gly Leu Arg Pro Gly Ala Gly Ala Glu His Trp Ser
145                 150                 155                 160

Tyr Gly Leu Arg Pro Gly Ala Gly Ala Glu His Trp Ser Tyr Gly Leu
                165                 170                 175

Arg Pro Gly Ala Gly Ala Glu His Trp Ser Tyr Gly Leu Arg Pro Gly
            180                 185                 190

Ala Arg Ala Arg Ala Val Gly Arg Pro Cys Pro Ile Cys Pro Ala Cys
        195                 200                 205

Glu Gly Pro Gly Pro Ser Ala Phe Ile Phe Pro Pro Lys Pro Lys Asp
    210                 215                 220

Thr Leu Met Ile Ser Arg Thr Pro Gln Val Thr Cys Val Val Val Asp
225                 230                 235                 240

Val Ser Gln Glu Asn Pro Glu Val Gln Phe Ser Trp Tyr Val Asp Gly
                245                 250                 255

Val Glu Val His Thr Ala Gln Thr Arg Pro Lys Glu Ala Gln Phe Asn
            260                 265                 270

Ser Thr Tyr Arg Val Val Ser Val Leu Pro Ile Gln His Glu Asp Trp
        275                 280                 285

Leu Lys Gly Lys Glu Phe Glu Cys Lys Val Asn Asn Lys Asp Leu Pro
    290                 295                 300

Ala Pro Ile Thr Arg Ile Ile Ser Lys Ala Lys Gly Pro Ser Arg Glu
305                 310                 315                 320

Pro Gln Val Tyr Thr Leu Ser Pro Ser Ala Glu Glu Leu Ser Arg Ser
                325                 330                 335

Lys Val Ser Ile Thr Cys Leu Val Thr Gly Phe Tyr Pro Pro Asp Ile
            340                 345                 350

Asp Val Glu Trp Lys Ser Asn Gly Gln Pro Glu Pro Glu Gly Asn Tyr
        355                 360                 365

Arg Thr Thr Pro Pro Gln Gln Asp Val Asp Gly Thr Tyr Phe Leu Tyr
    370                 375                 380

Ser Lys Leu Ala Val Asp Lys Ala Ser Trp Gln Arg Gly Asp Pro Phe
385                 390                 395                 400

Gln Cys Ala Val Met His Glu Ala Leu His Asn His Tyr Thr Gln Lys
                405                 410                 415

Ser Val Ser Lys Thr Gln Gly Lys His Asp Glu Leu
            420                 425

<210>    13
<211>    1565
<212>    DNA
<213>    Artificial Sequence

<220>
<223>    Full DNA sequence of 1300-BiP:CTB:GnRH:pFc2:HDEL

<400>    13
tctagaatta ttacatcaaa acaaaaaatg gctcgctcgt ttggagctaa cagtaccgtt      60

gtgttggcga tcatcttctt cggtgagtga ttttccgatc ttcttctccg atttagatct     120

cctctacatt gttgcttaat ctcagaacct tttttcgttg ttcctggatc tgaatgtgtt     180

tgtttgcaat ttcacgatct taaaaggtta gatctcgatt ggtattgacg attggaatct     240

ttacgatttc aggatgttta tttgcgttgt cctctgcaat agaagaggct acgaagttaa     300

ggatcctcac tcctcagaac ataaccgatt tgtgcgctga ataccataac actcaaatac     360

acacactcaa cgataagatt ttctcttaca ctgaatcact tgctggaaag agagagatgg     420

caattatcac ctttaaaaac ggagctactt tccaggttga agtgcctggt agtcaacata     480

tcgattcaca gaagaaagca atagagagga tgaaggatac acttaggatt gcttatttga     540

ccgaagcaaa agttgagaaa ctctgtgtgt ggaataacaa aacccctcac gctatcgctg     600

ctatctctat ggccactagt gcaggagcag aacattggtc ttacggtctt agacctggag     660

ctggagctga acattggagt tatggtttaa gacctggagc tggagctgaa cattggtctt     720

atggacttag accaggtgca ggtgctgagc attggtcata tggattgaga ccaggagctg     780

gtgctgagca ttggtcttat ggtctaagac ctggagctgg agctgaacat tggtcttacg     840

gactcagacc cggtgcccgg gcagttggaa gaccatgccc tatatgtcct gcttgtgaag     900

gtccaggtcc ctctgctttt atattcccac caaagccgaa ggataccttg atgatttcac     960

gtacaccaca agttacttgt gttgttgtgg atgtttcaca agaaaatcct gaggtacaat    1020

tcagctggta tgttgatggg gtagaagtgc acactgcaca gactcgacca aaggaggccc    1080

agtttaactc gacttataga gttgtttctg ttctcccaat ccaacacgaa gattggctga    1140

agggcaagga atttgaatgc aaggttaaca ataaagatct accagcacca attaccagga    1200

ttatttctaa ggcaaaagga ccctccagag agccccaagt ttacacattg tctccttctg    1260

ctgaggagct tagtagaagt aaagtgagca ttacctgctt agtgacggga ttctaccctc    1320

cagacatcga cgtcgaatgg aaatctaatg gtcaacctga gccagaaggt aactatagga    1380

ctactccacc acaacaggac gtcgatggca catactttct ttattcaaaa cttgctgtcg    1440

ataaggcaag ttggcaaaga ggagatccat ttcagtgtgc tgtaatgcat gaggctttgc    1500

ataatcatta tacacagaaa tcagtttcta aaacacaagg gaaacacgat gagctctagc    1560

tcgag                                                                1565

## Description

### [Technical Field]

The present invention relates to a vaccine composition for removing boar taint, and more particularly, to a recombinant protein for inducing antibodies against gonadotropin-releasing hormone (GnRH), a recombinant vector for producing the same, a vaccine composition for removing boar taint, which includes the recombinant protein as an active ingredient, a method for removing boar taint using the same, and the like.

This application claims priority to and the benefit of Korean Patent Application No. 10-2020-0035897 filed in the Korean Intellectual Property Office on March 24, 2020, and all the contents disclosed in the specification and drawings of that application are incorporated in this application.

### [Background Art]

Boar taint refers to the unpleasant odor or taste commonly found while people are cooking or eating pork or pork products, and although there is no problem of meat safety, the work of removing boar taint of boars is very important because the most important thing in the process of consumers choosing a meat is smell next to color.

The main causative materials of boar taint are skatole and androsterone, and skatole is a chemical material produced by the degradation of intestinal bacterial proteins and is also contained in human excreta. Skatole is degraded in the liver, but androsterone interferes with the degradation. That is, undegraded skatole accumulates especially in the fat part and disappears while dissipating into the air during the process of cooking pork, and this smell is just boar taint.

For centuries, pigs have been castrated to prevent boar taint, and castration of boars is performed about 2 to 3 weeks after birth. In some countries (the Netherlands, Switzerland, and Norway), general or local anesthetics may be used to reduce the pain and stress caused by castration, but recently, castration has been criticized by animal protection groups, whether pigs are anesthetized or not.

With the increasing criticism of the castration of boars, Australia and New Zealand began vaccination with Pfizer's commercial castration vaccine 'Improvac' in 1988, and the vaccine has been sold all over the world in places such as the USA, the EU, Japan, Switzerland, Australia and Brazil, and the product license was obtained in 2006 in Korea. An immunocastration vaccine may be a very safe and efficient solution which utilizes the porcine immune system to control boar taint. The vaccine stimulates the porcine immune system to produce specific antibodies against gonadotropin-releasing hormone (GnRH), thereby preventing causative materials of boar taint from being produced and accumulated by suppressing the function of the testes. In this regard, the expression "immunocastration" is used because the immunocastration uses the immune system to exhibit the same effect as that of castration which is a surgical procedure.

However, in Korea, because the removal of boar taint or improvement of meat quality by an immunocastration method does not meet the castration criteria 100% and the immunocastration method is very expensive compared to the surgical castration method (while the surgical castration method per head is 100 won, the immunocastration method per head is 5,000 won), the market entry of the corresponding vaccine has been unsuccessful.

Nonetheless, the immunocastration method not only removes boar taint, but also does not induce immunosuppression due to reduction in growth capacity, stress and pain which may appear after surgical castration, so that ultimately, there is a growing need for replacing the existing surgical castration method with the immunocastration method in order to aid in the improvement of the productivity of farmers using this immunocastration method and not to go against the global trend of animal welfare. Thus, there is a gradually increasing need for developing a vaccine for removing boar taint, which can be actually applied by overcoming the limitations of the existing immunocastration vaccine, such as high vaccination costs, uncertainty in grading, and safety issues and utilizing a purely domestic technology.

### [Disclosure]

### [Technical Problem]

The present invention has been devised to solve the technical problems in the related art as described above, and a vaccine including a recombinant protein for removing boar taint, which is fused with a carrier protein in order to artificially improve the antigenicity of gonadotropin-releasing hormone (GnRH) was developed, thereby completing the present invention.

Thus, an object of the present invention is to provide a vaccine composition for removing boar taint, the vaccine composition including, as an active ingredient, a recombinant protein in which a cholera toxin B subunit (CTB) and n gonadotropin-releasing hormones (GnRH) are fused, in which n is an integer from 1 to 20.

Another object of the present invention is to provide a method for removing the boar taint of an animal, the method including administering the vaccine composition according to the present invention to an animal except for a human.

Still another object of the present invention is to provide a recombinant vector for removing boar taint, the recombinant vector including a GnRH gene including a base sequence of SEQ ID NO: 3 and a CTB gene including a base sequence of SEQ ID NO: 5.

Yet another object of the present invention is to provide a recombinant protein for removing boar taint produced using the recombinant vector according to the present invention.

Still yet another object of the present invention is to provide a transformant transformed with the recombinant vector according to the present invention.

However, the technical problems which the present invention intends to solve are not limited to the technical problems which have been mentioned above, and other technical problems which have not been mentioned will be apparently understood by a person with ordinary skill in the art to which the present invention pertains from the following description.

### [Technical Solution]

In order to achieve the objects of the present invention as described above, the present invention provides a vaccine composition for removing boar taint, the vaccine composition including, as an active ingredient, a recombinant protein in which a cholera toxin B subunit (CTB) and n gonadotropin-releasing hormones (GnRH) are fused, in which n is an integer from 1 to 20.

Further, the present invention provides a method for removing boar taint by administering, to an individual except for a human, a vaccine composition including, as an active ingredient, a recombinant protein in which CTB and n GnRHs are fused, in which n is an integer from 1 to 20.

In addition, the present invention provides a use of a recombinant protein in which CTB and n GnRHs are fused, in which n is an integer from 1 to 20 for removing boar taint.

Furthermore, the present invention provides a use of a recombinant protein in which CTB and n GnRHs are fused, in which n is an integer from 1 to 20, for producing a vaccine used to remove boar taint.

In an exemplary embodiment of the present invention, the GnRH may include an amino acid sequence of SEQ ID NO: 1, but is not limited thereto.

In another exemplary embodiment of the present invention, the CTB may include an amino acid sequence of SEQ ID NO: 4, but is not limited thereto.

In still another exemplary embodiment of the present invention, n may be 6, but is not limited thereto.

In yet another exemplary embodiment of the present invention, when n is 6, the GnRH may include an amino acid sequence of SEQ ID NO: 2, but is not limited thereto.

In still yet another exemplary embodiment, when n is 2 or higher, the GnRHs may be linked to each other via an alanine-glycine-alanine linker, but are not limited thereto.

In an exemplary embodiment of the present invention, the recombinant protein may be further fused with a porcine immunoglobulin Fc fragment (pFc2).

In another exemplary embodiment of the present invention, the pFc2 may include an amino acid sequence of SEQ ID NO: 6, but is not limited thereto.

In still another exemplary embodiment of the present invention, the CTB may be fused in the N-terminal direction of GnRH and the pFc2 may be fused in the C-terminal direction of GnRH, but are not limited thereto.

In an exemplary embodiment of the present invention, the recombinant protein may be further fused with a chaperone binding protein (BiP).

In another exemplary embodiment of the present invention, the BiP may include an amino acid sequence of SEQ ID NO: 8, but is not limited thereto.

In still another exemplary embodiment of the present invention, the CTB may be fused in the N-terminal direction of GnRH and the BiP may be fused in the N-terminal direction of CTB, but are not limited thereto.

In an exemplary embodiment of the present invention, the recombinant protein may be further fused with His-Asp-Glu-Leu (HDEL).

In another exemplary embodiment of the present invention, the HDEL may be encoded by a base sequence of SEQ ID NO: 11, but is not limited thereto.

In still another exemplary embodiment of the present invention, the CTB may be fused in the N-terminal direction of GnRH and the HDEL may be fused in the C-terminal direction of GnRH, but are not limited thereto.

Further, in an exemplary embodiment of the present invention, the recombinant protein may be a recombinant protein in which BiP, CTB, GnRH, pFc2, and HDEL are sequentially fused, but is not limited thereto.

In another exemplary embodiment of the present invention, the recombinant protein may include an amino acid sequence of SEQ ID NO: 12, but is not limited thereto.

In an exemplary embodiment of the present invention, the vaccine composition may further include an adjuvant.

In another exemplary embodiment of the present invention, the adjuvant may be an Emulsigen-based adjuvant, but is not limited thereto.

In addition, the present invention provides a method for immunocastration of an animal, the method including: administering the vaccine composition according to the present invention to an animal except for a human.

In an exemplary embodiment of the present invention, the animal may be a dog, a whale, a cat, a gorilla, a goose, a guinea pig, a pheasant, a camel, a roe deer, a mule, a chicken, a donkey, a dolphin, a pig, a llama, a horse, a water buffalo, a deer, a cow, a reindeer, an alpaca, a yak, a sheep, a goat, an orangutan, a duck, a monkey, a ferret, a rat, a turkey, an ostrich, or a rabbit, but is not limited thereto.

Furthermore, the present invention provides a recombinant vector for removing boar taint, the recombinant vector including a GnRH gene including a base sequence of SEQ ID NO: 3 and a CTB gene including a base sequence of SEQ ID NO: 5.

In an exemplary embodiment of the present invention, the recombinant vector may further include a polynucleotide encoding pFc2 including an amino acid sequence of SEQ ID NO: 6, but is not limited thereto.

In another exemplary embodiment of the present invention, the recombinant vector may further include a polynucleotide encoding BiP including an amino acid sequence of SEQ ID NO: 8, but is not limited thereto.

In still another exemplary embodiment of the present invention, the recombinant vector may further include an HDEL gene including a base sequence of SEQ ID NO: 11, but is not limited thereto.

In yet another exemplary embodiment of the present invention, the recombinant vector may include a polynucleotide encoding an amino acid sequence of SEQ ID NO: 12, or a base sequence of SEQ ID: 13, but is not limited thereto.

Further, the present invention provides a recombinant protein for removing boar taint produced using the recombinant vector according to the present invention.

In addition, the present invention provides a transformant transformed with the recombinant vector according to the present invention.

### [Advantageous Effects]

The present invention relates to a vaccine composition for removing boar taint, the vaccine composition including a recombinant in which a cholera toxin B subunit (CTB) and gonadotropin-releasing hormone (GnRH) are fused, and using a recombinant protein including a polypeptide sequence of GnRH, antibodies against GnRH were induced in an individual to which the recombinant protein was administered, whereby the effect of atrophying the testes was confirmed. Therefore, the present invention can be usefully used to remove boar taint by immunologically castrating a boar at low cost and with high safety and minimal side effects.

### [Description of Drawings]

FIG. 1 is a view illustrating an expression cassette for producing a recombinant GnRH protein according to an exemplary embodiment of the present invention (BiP, chaperone binding protein; CTB, Cholera toxin B subunit; pFc2, porcine Fc fragment; HDEL, Histidine-Aspartic acid-Glutamic acid-Leucine).
FIG. 2 is a view illustrating the result of isolating and purifying the recombinant GnRH protein according to an exemplary embodiment of the present invention.
FIG. 3 is a view illustrating a schedule of administering a vaccine composition including the recombinant GnRH protein according to an exemplary embodiment of the present invention to male rats.
FIG. 4 is a view comparing the sizes of the testes excised after administration of the vaccine composition including the recombinant GnRH protein according to an exemplary embodiment of the present invention to male rats three times.
FIG. 5 is a view illustrating the results of measuring the concentration of blood testosterone after administration of the vaccine composition including the recombinant GnRH protein according to an exemplary embodiment of the present invention to male rats three times, the upper and middle drawings are views illustrating a standard curve, and the lower drawing is a view illustrating the measurement results of each group.

### [Modes of the Invention]

The present inventors inoculated 4-week-old rats with a recombinant gonadotropin-releasing hormone (GnRH) protein, and then analyzed the change in size and weight of the testes and the change in blood testosterone concentration according to the inoculation. As a result, by confirming that a group vaccinated with the recombinant GnRH protein of the present invention had a decrease in size and weight of testes and a decrease in blood testosterone concentration as compared to the control, the present inventors confirmed that a vaccine composition including the recombinant GnRH protein according to the present invention has an immunocastration effect and an accompanying boar taint removal effect, thereby completing the present invention.

Thus, the present invention may provide a vaccine composition for removing boar taint, the vaccine composition including, as an active ingredient, a recombinant protein in which a cholera toxin B subunit (CTB) and n gonadotropin-releasing hormones (GnRH) are fused, in which n is an integer from 1 to 20.

As used herein, the term "cholera toxin B subunit (CTB)" refers to a polypeptide having binding activity to host cells, is preferably encoded by a polynucleotide including a base sequence of SEQ ID NO: 5, is most preferably encoded by a polynucleotide represented by SEQ ID NO: 5, but may be encoded by a base sequence having a sequence homology 80% or more, more preferably 90% or more, and even more preferably 95% or more with the base sequence of SEQ ID NO: 5. For example, the cholera toxin B subunit includes a polypeptide having a sequence homology of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. The % sequence homology to a polynucleotide is confirmed by comparing a comparison region with an optimally aligned sequence, and a portion of the polynucleotide sequence in the comparison region may further include an addition or deletion (that is, gap) compared to the reference sequence (without addition or deletion) for the optimal alignment of the sequence. Cholera toxin is a complex in a form in which A1 and A2 subunits and five B subunits are bound to one another, and the CTB binds to microfold cells (M cells) of the mucosal immune system for internalization and activation of cholera toxin. In this case, the CTB specifically reacts with and binds to GM1-ganglioside of the cell membrane bilayer, and then the A subunit or antigen protein bound to CTB passes through the cell membrane. The CTB may be a strong mucosal adjuvant and carrier molecule for enhancing mucosal immunity by increasing the absorption of a binding antigen passing through the mucosal barrier and efficiently presenting the binding antigen to improve the immunogenicity of the antigen.

As used herein, the term "polynucleotide" refers to an oligomer or polymer containing two or more linked nucleotides or nucleotide derivatives generally bound to each other via a phosphodiester bond, including deoxyribonucleic acid (DNA) and ribonucleic acid (RNA). The polynucleotide also includes DNA and RNA derivatives including, for example, a nucleotide analog or a backbone bond other than a phosphodiester bond, for example, a phosphotriester bond, a phosphoramidate bond, a phophorothioate bond, a thioester bond, or a peptide bond (peptide nucleic acid). The polynucleotide includes single-stranded and/or double-stranded polynucleotides, for example, deoxyribonucleic acid (DNA) and ribonucleic acid (RNA) as well as analogs of either RNA or DNA.

As used herein, the term "gonadotropin-releasing hormone (GnRH)" refers to a gonadotropin-releasing hormone, and is preferably GnRH including an amino acid sequence of SEQ ID: 1, but is not limited as long as an immune response can be induced against GnRH. Further, the GnRH of the present invention includes a variant of SEQ ID NO: 1 within the scope of the present invention. Specifically, the peptide may include an amino acid sequence having a sequence homology of 80% or more, more preferably 90% or more with an amino acid sequence of SEQ ID NO: 1. The GnRH consists of 10 amino acids, and is known to consist of almost the same amino acids in all animals. The GnRH is produced in the hypothalamus and acts on the pituitary gland to promote the production of the sex hormones FSH and LH, and when the GnRH is used as an antigen to inoculate an animal, an antibody which recognizes the GnRH is formed, and sexual maturation of male and female animals is blocked by removing GnRH present in the body through the antibody, so that a castration effect occurs, and as a result, an effect of removing boar taint is exhibited.

As used herein, the term "recombinant protein" or "fused recombinant protein" refers to a protein in which two or more proteins are artificially linked, and refers to a protein in a form where the CTB and the GnRH are linked to each other in the present invention. The fused recombinant protein as described above may be chemically synthesized after each partner is determined, or may be obtained by expressing and purifying the recombinant protein by a gene recombination method. Preferably, the fused recombinant protein may be obtained by expressing a fused gene which links a polynucleotide encoding the CTB to a polynucleotide encoding the GnRH in a cell expression system.

It is preferred that the GnRH used in the recombinant protein of the present invention has two or more copies of a GnRH amino acid sequence rather than one copy of the GnRH amino acid sequence. When the GnRH has such multiple copies, immunogenicity against GnRH may be further enhanced, so that an amino acid sequence of GnRH, preferably an amino acid sequence of SEQ ID NO: 1 may take two more multimers. The number of GnRHs is not particularly limited as long as the GnRH can be accepted in an expression vector, but the GnRH may take a multimer form in which preferably two to 20 GnRHs, more preferably 2 to 10 GnRHs are linked, and even more preferably, a hexamer in which 6 GnRHs are linked may be used. In a specific example of the present invention, the GnRH protein was constructed so as to be repeated 6 times in a row. The polypeptide in which 6 GnRHs are linked may include an amino acid sequence of SEQ ID NO: 2, but is not limited thereto.

Further, in such a fusion recombinant protein, CTB and GnRH may be linked directly or via an amino acid connector such as a linker. In addition, even when several copies of GnRH are included, each GnRH may be linked directly or via a connector such as a linker. When GnRH is linked via a linker, it is preferred that the entire immunogenicity is not reduced thereby.

As used herein, the term "linker" refers to a peptide to be inserted between proteins, such that when a recombinant protein is produced by linking CTB to GnRH, or several GnRHs are linked, the activity of each protein bound by increasing the structural flexibility of these proteins may be enhanced. The type and the number of amino acids of the linker are not limited as long as the linker can minimize the immune response, but 1 to 20 amino acids are preferred, and 1 to 5 amino acids are more preferred. In a specific example of the present invention, the sequence of a restriction enzyme site at the multiple cloning site of the vector was used, or an alanine-glycine-alanine (AGA) linker was used in order to link each protein.

As used herein, the term "vaccine" is a biological preparation containing an antigen that causes an immune response in an organism, and refers to an immunogen that induces immunity in an organism by injection or oral administration into a human or animal to prevent an infectious disease or induce an antibody against a particular antigen. The animal is a non-human animal, examples of which are described below.

The vaccine composition of the present invention may be used being formulated in the form of an oral formulation such as a powder, a granule, a tablet, a capsule, a suspension, an emulsion, a syrup, and an aerosol, and a sterile injection solution, according to a typical method. When the composition is prepared, the composition may be prepared using a commonly used diluent or excipient, such as a filler, an extender, a binder, a wetting agent, a disintegrant, and a surfactant. A solid formulation for oral formulation includes a tablet, a pill, a powder, a granule, and the like, and the solid formulation may be prepared by mixing at least one excipient, for example, starch, calcium carbonate, sucrose or lactose, gelatin, and the like with a lecithin-like emulsifier. Further, in addition to simple excipients, lubricants such as magnesium stearate and talc may also be used. As a liquid formulation for oral administration, a suspension, a liquid for internal use, an emulsion, a syrup, and the like may be used, and in addition to water and liquid paraffin which are simple commonly used diluents, various excipients, for example, a wetting agent, a sweetener, an aromatic, a preservative, and the like may be included. Examples of a formulation for parenteral administration include an aqueous sterile solution, a non-aqueous solvent, a suspension, an emulsion, and a freeze-dried preparation. As the non-aqueous solvent and the suspension, it is possible to use propylene glycol, polyethylene glycol, a vegetable oil such as olive oil, an injectable ester such as ethyl oleate, and the like.

The route of administration of the vaccine composition according to the present invention includes, but is not limited to, oral, intravenous, intramuscular, intraarterial, intramedullary, intrathecal, intracardiac, transdermal, subcutaneous, intraperitoneal, intranasal, intestinal canal, topical, sublingual or rectal routes. Oral or parenteral administration is preferred. As used herein, the term "parenteral" includes subcutaneous, intradermal, intravenous, intramuscular, intraarticular, intrasynovial, intrasternal, intrathecal, intralesional and intracranial injection or infusion techniques. A vaccine composition of the present invention may also be administered in the form of a suppository for rectal administration, but is not limited to the administration methods listed above, and may be administered by any device capable of moving an active material to target cells. The vaccine composition according to the present invention may induce a humoral or selectively cell-mediated response and/or a combination of the two immune responses.

The dose of the vaccine composition according to the present invention is selected in consideration of the age, body weight, sex, physical condition and the like of an individual. The amount required to induce an immune response in an individual without particular side effects may vary depending on the recombinant protein used as an immunogen and any presence of an excipient.

Meanwhile, a vaccine composition for immunocastration according to the present invention may include an antigen, a pharmaceutically acceptable carrier, an appropriate adjuvant, and other typical materials, and is administered in an immunologically effective amount. As used herein, the term "immunologically effective amount" refers to a sufficient amount capable of exhibiting an immunocastration effect and an amount which does not cause side effects or severe or excessive immune responses, and the exact dose concentration will depend on a particular immunogen to be administered and can be determined by those skilled in the art using methods known to examine the generation of an immune response. Further, the immunologically effective amount may vary depending on the dosage form and route, the age, health and body weight of a recipient, the characteristics and extent of symptoms, the type of current therapy, and the number of treatments.

In addition, a recombinant protein according to the present invention may be further fused with a porcine immunoglobulin Fe fragment (pFc2). The pFc2 may be fused in the C-terminal direction of GnRH, but is not limited thereto.

As used herein, the Fc fragment refers to a portion which is linked only to a heavy chain (H chain) portion by an S-S bond and does not have any antigen binding site when immunoglobulin is digested by papain, and the Fc fragment of the present invention is preferably a porcine Fc fragment, and more preferably a porcine Fc fragment including an amino acid sequence including SEQ ID No. 6, but is not limited as long as an Fc fragment increases the expression level and solubility of a recombinant protein, when fused with the recombinant protein. Further, the Fc fragment of the present invention includes a variant of SEQ ID No. 6 within the scope of the present invention. Specifically, the peptide may include an amino acid sequence having a sequence homology of 90% or more, more preferably 95% or more, and most preferably 98% or more with an amino acid sequence of SEQ ID NO: 6.

In addition, the recombinant protein according to the present invention may be further fused with a chaperone binding protein (BiP). The BiP may be fused in the N-terminal direction of GnRH or CTB, but is not limited thereto.

As used herein, the term "chaperone binding protein (BiP)" is used to move an expressed recombinant protein to the endoplasmic reticulum, but is not limited as long as a Bip moves a recombinant protein to the endoplasmic reticulum when fused with the recombinant protein. Furthermore, the BiP of the present invention includes a variant of SEQ ID NO: 8 within the scope of the present invention. Specifically, the peptide may include an amino acid sequence having a sequence homology of 90% or more, more preferably 95% or more, and most preferably 98% or more with an amino acid sequence of SEQ ID NO: 8. When or after the BiP is expressed, a part of the peptide may be cut off, so that only a part of the peptide may remain

Further, the recombinant protein according to the present invention may be further fused with His-Asp-Glu-Leu (HDEL). The HDEL may be fused in the C-terminal direction of GnRH or pFc2, but is not limited thereto.

As used herein, the term "HDEL" refers to a target peptide sequence including histidine-aspartic acid-glutamic acid-leucine (HDEL), and the HDEL prevents a recombinant protein from being secreted from the endoplasmic reticulum or allows the recombinant protein to be maintained in the endoplasmic reticulum. The HDEL may be encoded by a base sequence of SEQ ID NO: 11, but is not limited thereto.

In addition, the recombinant protein according to the present invention may be a recombinant protein in which BiP, CTB, GnRH, pFc2, and HDEL are sequentially fused, and the resulting recombinant protein may be a recombinant protein including an amino acid sequence of SEQ ID NO: 12, but the present invention is not limited by the fusion sequence or amino acid sequence.

The vaccine composition according to the present invention has an excellent effect of removing boar taint.

Furthermore, the vaccine composition according to the present invention may include one or more adjuvants to improve or enhance the immune response.

As used herein, the term "adjuvant" refers to a material or composition which may be added to a vaccine or pharmaceutically active components to increase or affect the immune response. Representatively, the adjuvant typically refers to a carrier or auxiliary material for an immunogen and/or another pharmaceutically active material or composition. Typically, the term "adjuvant" should be interpreted as a broad concept, and refers to a material or stratagem in a broad range, which may enhance the immunogenicity of an antigen which is integrated into the adjuvant or administered with the adjuvant. Further, the adjuvant is not limited thereto, and may be divided into an immune potentiator, an antigen delivery system, or a combination thereof. Examples of an appropriate adjuvant may include aluminum hydroxide, a Freund's complete or incomplete adjuvant, DEAE dextran, levamisole, PCG, and poly I:C or poly A:U. In an exemplary embodiment of the present invention, an Emulsigen-D adjuvant including dimethyldioctadecyl ammonium bromide (DDA) was used as an Emulsigen-based adjuvant.

The recombinant protein according to the present invention includes not only a protein having a wild type amino acid sequence, but also a variant of a fused recombinant protein. The variant of the recombinant protein refers to a protein having a normal amino acid sequence and a different sequence in which one or more amino acid residues undergo deletion, insertion, non-conservative or conservative substitution, or a combination thereof. Amino acid exchanges at the protein and peptide levels that do not alter the activity of the molecule as a whole are known in the art (H. Neurath et al., 1979). In addition, the recombinant protein may also be modified with phosphorylation, methylation, sulfation, acrylation, glycosylation, farnesylation, and the like. The variant or modifier is a functional equivalent showing the same biological activity as that of a natural protein, but may be a variant or modifier in which characteristics of the protein thereof are modified, if necessary. Preferably, the structural stability of a protein against heat, pH, and the like is increased or the protein activity is increased by the variation and modification in an amino acid sequence.

A recombinant GnRH protein according to the present invention may be chemically synthesized or produced by a gene recombination method, and may be preferably produced using a recombinant vector to transform a host cell with the recombinant vector and isolate and purify a protein expressed from the transformed host cell.

Accordingly, as another aspect of the present invention, the present invention may provide a recombinant vector for removing boar taint, the recombinant vector including a GnRH gene including a base sequence of SEQ ID NO: 3 and a CTB gene including a base sequence of SEQ ID NO: 5.

The recombinant vector is typically a carrier into which a fragment of a foreign DNA is inserted, and generally refers to a fragment of a double-stranded DNA. As used herein, the term "foreign DNA" refers to DNA which originates from a foreign species, or refers to a form which is substantially modified from the original form when the foreign DNA originates from the same species, and the foreign gene encodes a polypeptide as a nucleic acid of particular interest to be transcribed. A recombinant vector should be operably linked to a transcriptional and translational expression regulatory sequence which exhibits functions within an expression host in which the corresponding gene is selected in order to enhance the expression level of a transformed gene in a host cell. The recombinant vector is a gene construct including an essential regulatory element operably linked such that a gene insert is expressed in the cells of an individual, and a standard recombinant DNA technique may be used in order to prepare these gene constructs. The type of recombinant vector is not particularly limited as long as the recombinant vector expresses a target gene in various host cells such as prokaryotic cells and eukaryotic cells and functions to produce the target protein, but a vector capable of mass-producing a foreign protein having a form similar to the natural state while having a promoter having strong activity and strong expression power is preferred. It is preferred that the recombinant vector includes at least a promoter, a start codon, a gene encoding a target protein, a stop codon, and a terminator. In addition, the recombinant vector may also appropriately include DNA encoding a signal peptide, an enhancer sequence, an untranslated region (UTR) at the 5' end and 3' end of a target gene, a selection marker region, a replicable unit, or the like

In specific embodiments of the present invention, a tobacco plant (*Nicotiana benthamiana*) was transformed with a recombinant vector prepared by inserting a CTB gene fragment into a PCAMBIA13000 vector including 6 copies of the GnRH gene fragment. The vector has a 35S promoter and a heat shock protein (HSP) terminator system, and is a vector useful for mass-producing proteins in tobacco plants.

As used herein, the term "vector" refers to a DNA preparation containing a DNA sequence operably linked to a suitable regulatory sequence capable of expressing the DNA in a suitable host. The vector may be a plasmid, a phage particle, or simply a potential genomic insert. Once transformed into a suitable host, the vector may be replicated and function independently of the host genome, or may be integrated into the genome itself in some cases. Since the plasmid is the most commonly used type of current vector, the terms plasmid and vector may be sometimes used interchangeably. However, the present invention includes other forms of known vectors or having functions equivalent to those known in the art.

Further, in the present invention, the recombinant vector may further include one or more sequences selected from the group consisting of a polynucleotide encoding pFc2 including an amino acid sequence of SEQ ID NO: 6, a polynucleotide encoding BiP including an amino acid sequence of SEQ ID NO: 8, and an HDEL gene including a base sequence of SEQ ID NO: 11.

As still another aspect of the present invention, the present invention may provide a recombinant protein for removing boar taint produced using the recombinant vector according to the present invention.

Further, as yet another aspect of the present invention, the present invention may provide a transformant transformed with the recombinant vector according to the present invention.

As used herein, the term "transformation" is a generic term which refers to changes in genetic properties of an organism due to an injected DNA, a transformant is an organism produced by injecting a foreign gene by a molecular genetic method, and is preferably an organism transformed by the recombinant vector of the present invention, and the organism is not limited as long as the organism achieves the object of the present invention.

As still yet another aspect of the present invention, the present invention may provide a method for removing boar taint, the method including administering the vaccine composition according to the present invention to an animal except for a human.

The method for removing boar taint using the vaccine composition of the present invention is applicable to all animals except for a human. The animal is preferably a mammal, and may be exemplified by a dog, a whale, a cat, a gorilla, a goose, a guinea pig, a pheasant, a camel, a roe deer, a mule, a chicken, a donkey, a dolphin, a pig, a llama, a horse, a water buffalo, a deer, a cow, a reindeer, an alpaca, a yak, a sheep, a goat, an orangutan, a duck, a monkey, a ferret, a rat, a turkey, an ostrich, or a rabbit, but is not limited thereto.

In the method of the present invention, it is preferred that the administration is performed twice or more. For example, after the initial vaccination, booster injections may be performed 1 to 4 times at intervals of 1 to 10 weeks, but booster injections may be appropriately modified and performed by those skilled in the art depending on the type of corresponding animal.

Terms or words used in the specification and the claims should not be interpreted as being limited to a typical or dictionary meaning and should be interpreted as a meaning and a concept which conform to the technical spirit of the present invention based on a principle that an inventor can appropriately define a concept of a term in order to describe his/her own invention by the best method.

Hereinafter, preferred examples for helping the understanding of the present invention will be suggested. However, the following examples are provided only to more easily understand the present invention, and the contents of the present invention are not limited by the following examples.

### [Examples]

### Example 1: Expression and purification of BiP-CTB-GnRH-pFc2-HDEL fusion protein

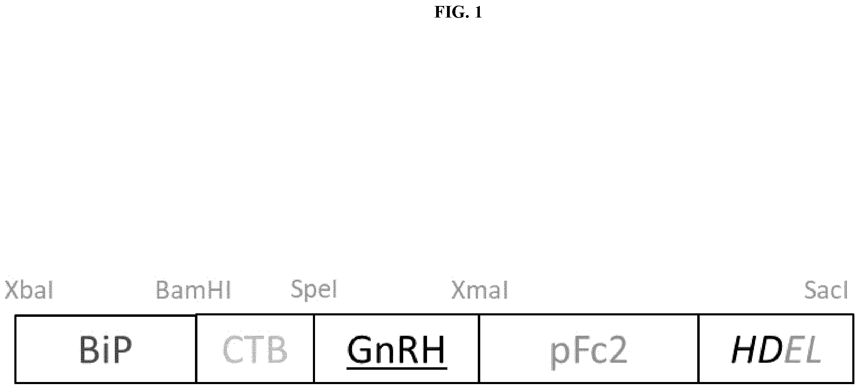

In order to induce immunogenicity against GnRH, as illustrated in FIG. 1, a vector for expressing recombinant GnRH in which CTB, pFc2, BiP, and HDEL were fused with GnRH was constructed. GnRH including 10 amino acids was linked by an alanine-glycine-alanine (AGA) linker, such that 6 GnRHs were continuous. The amino acid sequence and base sequence of 6 linked GnRHs are shown in SEQ ID NO: 2 and SEQ ID NO: 3, respectively. Further, the amino acid sequence and base sequence for CTB are shown in SEQ ID NO: 4 and SEQ ID NO: 5, respectively, the amino acid sequence and base sequence for pFc2 are shown in SEQ ID NO: 6 and SEQ ID NO: 7, respectively, the amino acid sequence and base sequence for BiP are shown in SEQ ID NO: 8 and SEQ ID NO: 9, respectively, the amino acid sequence and base sequence for HDEL are shown in SEQ ID NO: 10 and SEQ ID 11, and the entire amino acid sequence and base sequence of an expression cassette are shown in SEQ ID NO: 12 and SEQ ID NO: 13, respectively.

Leaves of a tobacco plant were inoculated with *Agrobacterium tumefaciens* transformed with the vector for transient expression. After the total protein was extracted from the leaves of the plant and centrifuged, a pFc2 fusion GnRH protein was isolated as a soluble form of the protein included in the solution using AKTA prime (GE Healthcare, USA) equipped with a protein A-sepharose column.

### Example 2: Confirmation of concentration of recombinant GnRH protein

In order to confirm and measure the concentration of the recombinant GnRH protein isolated and purified in Example 1, SDS-PAGE and a Bradford assay were performed using bovine serum albumin (BSA) having a known concentration as a standard. 1 and 2 µl of the isolated and purified recombinant GnRH protein were loaded, and 0.5, 1, and 2 µg of BSAwere included for quantification. For Gel, a band was finally confirmed by Coomassie blue staining. For the Bradford assay, a calculation equation was made using BSA as a standard sample to draw a standard curve, and the concentration was calculated by substituting the UV value of GnRH.

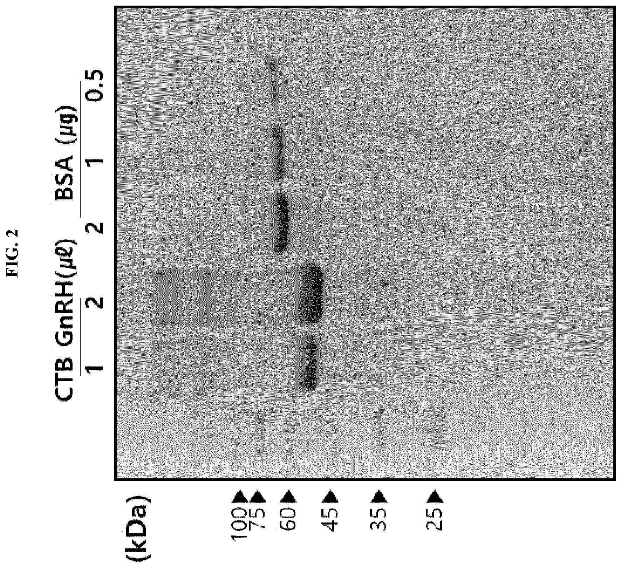

As a result, as illustrated in FIG. 2, it was confirmed that the recombinant GnRH protein was well expressed, isolated and purified, and it was confirmed that the concentration of the GnRH recombinant protein was about 2 µg/µl. The recombinant GnRH protein confirmed as described above was used in an animal experiment of the following Example 3.

### Example 3: Confirmation of effect of administering recombinant GnRH protein

### 3.1. Experimental animals, administration conditions, and schedule

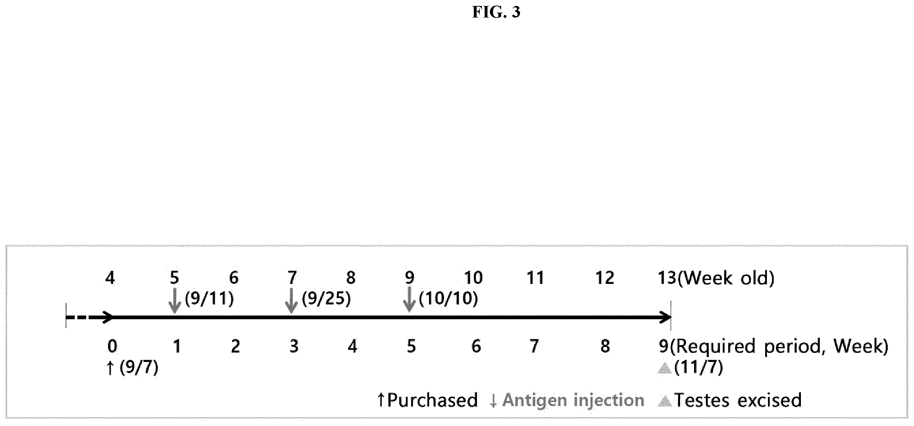

In order to confirm the immunocastration effect of the recombinant GnRH protein, a vaccine composition including the recombinant GnRH protein according to the present invention was administered to 4-week-old male Sprague Dawley (SD) rats under the administration conditions shown in the following Table 1 according to the schedule shown in FIG. 3. The same amount of phosphate-buffered saline (PBS) was administered to a control and 100 µg/ 180 µl of the vaccine composition and 20 µl of an Emulsigen-D adjuvant were administered to an experimental group (see the following Table 1). After the 4-week-old rats, which had been purchased, were stabilized for about 4 days, administration was performed three times in total at an administration interval of 14 to 15 days. Blood was collected before the administration and after the completion of the experiment, the testes were excised to measure the weight and the size at the time point of 4 weeks after the third administration, and the concentration of blood testosterone was measured.

**[Table 1]**

| | | Control | Experimental group (recombinant GnRH protein) |
|---|---|---|---|
| Administra tion | Antigen | PBS 180 µl | 100 µg / 180 µl |
| | Adjuvant | 20 µl | 20 µl |
| Experimental population | | 3 | 3 |

### 3.2. Visual evaluation of testes

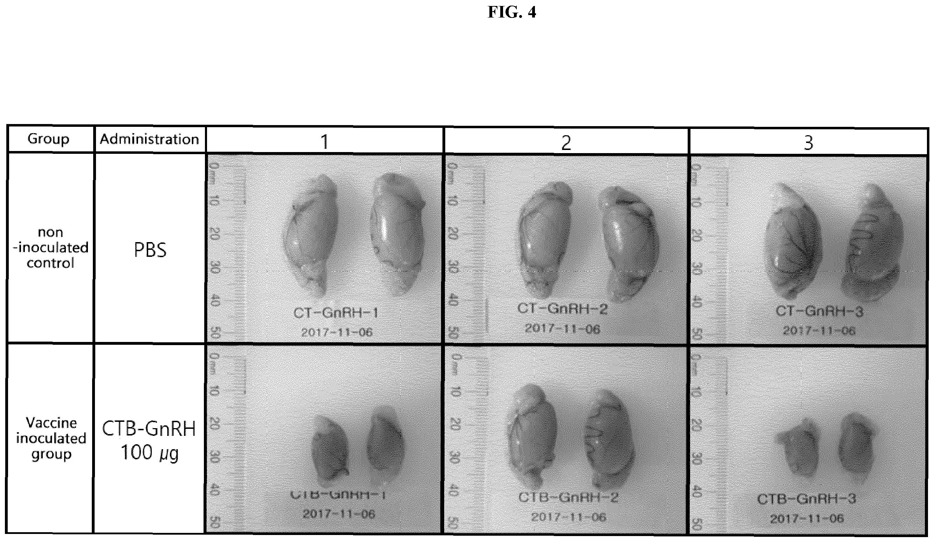

At the time point of 4 weeks after the third administration of the vaccine composition according to the present invention, the testes were excised and visually evaluated. The results are shown in the following Table 2 and FIG. 4.

**[Table 2]**

| Group | Adminis tration | Indivi dual | Testis | | Body weight (g) | Weight ratio | Length ratio |
|---|---|---|---|---|---|---|---|
| | | | Weight (g) | Length (mm) | | (%)** | (%)*** |
| Non-inoculated control | PBS | 1 | 2.90 | 37.5 | 461.7 | 0.63 | 103.31 |
| | | 2 | 2.59 | 35.5 | 483.7 | 0.54 | 97.80 |
| | | 3 | 2.52 | 36.0 | 443.2 | 0.57 | 99.17 |
| Vaccine inoculated group | CTB-GnRH 100 *µ*g | 1 | 0.74 | 23.5 | 396.5 | 0.19 | 64.74 |
| | | 2 | 1.98 | 31.5 | 458.1 | 0.43 | 86.78 |
| | | 3 | 0.60 | 22.0 | 383.2 | 0.16 | 60.61 |
| * Weight, length: Left and right average of testes (including epididymis) | | | | | | | |
| ** Weight ratio (%) = Average weight of testes of each individual/Total body weight of each individual × 100% | | | | | | | |
| *** Length ratio (%) = Average length of testes of each individual/Average length of testes of control × 100% | | | | | | | |

As shown in Table 2 and FIG. 4, it was confirmed that the weights and sizes of the testes of a vaccine inoculated group including the recombinant GnRH protein were significantly decreased as compared to those of a non-inoculated control. Further, as can be confirmed by the weight ratio, it was found that the ratio of the weight of the testes to the total body weight of an individual was remarkably low in the vaccine inoculated group, and it was also found that the average length of the testes of the vaccine inoculated group was 64% to 86% of that of the non-inoculated group.

The results as described above confirm that immunocastration was successfully achieved by the administration of the vaccine composition including the recombinant GnRH protein according to the present invention.

### 3.3. Testosterone ELISA results

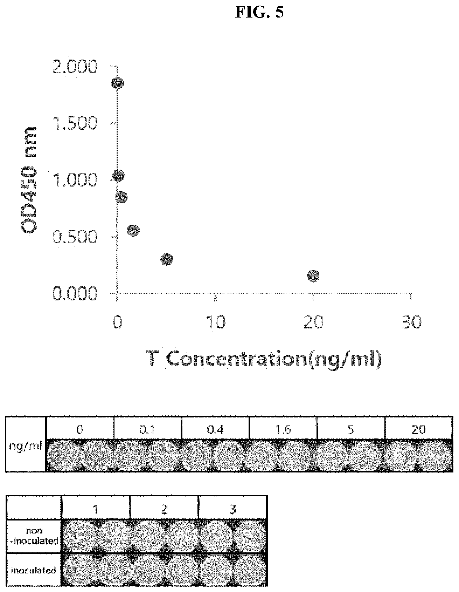

At the time point of 4 weeks after the third administration of the vaccine composition according to the present invention, the concentration of blood testosterone was measured by collecting blood. The measurement was performed using an ELISA kit of Elabscience according to the manufacturer's protocol. Specifically, 50 µL of serum was put into a plate coated with testosterone, and exactly the same amount of a biotinylated detection antibody was put into the plate. After the resulting mixture was reacted at 37°C for 45 minutes, the plate was washed three times using 350 µL of a washing buffer. Subsequently, 100 µL of an HRP conjugate solution was added thereto and the resulting mixture was cultured at 37°C for 30 minutes. After washing was performed 5 times in the same manner as in the above-described procedure, 90 µL of a substrate solution was added thereto, and then the resulting mixture was cultured at 37°C for 15 minutes. Thereafter, 50 µL of a reaction stop solution was added thereto, and the OD value was measured at a wavelength of 450 nm by an ELISA machine. In order to quantify the actual amount of blood testosterone, the reaction was carried out by including a testosterone standard protein at a concentration of 0.1, 0.4, 1.6, 5, and 20 ng/ml. A standard curve was made with the OD values obtained through the process, and testosterone was quantified by substituting the OD values of the samples previously obtained into the equation of the curve. The results are shown in the following Table 3 and FIG. 5.

**[Table 3]**

| | | | | | | |
|---|---|---|---|---|---|---|
| Standard (ng/ml) | OD450 nm | | Group | Administration | Individ ual | OD450 nm |
| 0 | 1.852 | | Non-inoculated control | PBS | 1 | 0.171 |
| 0.1 | 1.037 | | | | 2 | 0.150 |
| 0.4 | 0.850 | | | | 3 | 0.194 |
| 1.6 | 0.559 | | Vaccine inoculated group | CTB-GnRH 100 µg | 1 | 0.491 |
| 5 | 0.302 | | | | 2 | 0.540 |
| 20 | 0.154 | | | | 3 | 0.530 |

As a result, as shown in Table 3 and FIG. 5, it was found that the concentration of blood testosterone of the vaccine inoculated group including the recombinant GnRH protein was remarkably decreased as compared to that of the non-inoculated control.

The result as described above coincides with the result in Example 3.2., confirming that immunocastration was successfully achieved by the administration of the vaccine composition including the recombinant GnRH protein according to the present invention, and accordingly, boar taint was removed.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

The present invention relates to a vaccine composition for removing boar taint, the vaccine composition including a recombinant in which a cholera toxin B subunit (CTB) and gonadotropin-releasing hormone (GnRH) are fused, and using a recombinant protein including a polypeptide sequence of GnRH, antibodies against GnRH were induced in an individual to which the recombinant protein was administered, whereby the atrophy effect of the testes was confirmed. Therefore, the present invention can be used to remove boar taint by immunologically castrating a boar at low cost and with high safety and minimal side effects, and thus is expected to have high industrial applicability.

## Claims

1. A vaccine composition for removing boar taint, the vaccine composition comprising, as an active ingredient, a recombinant protein in which a cholera toxin B subunit (CTB) and n gonadotropin-releasing hormones (GnRH) are fused,
wherein n is an integer from 1 to 20.

2. The vaccine composition of claim 1, wherein the GnRH comprises an amino acid sequence of SEQ ID NO: 1.

3. The vaccine composition of claim 1, wherein the CTB comprises an amino acid sequence of SEQ ID NO: 4.

4. The vaccine composition of claim 1, wherein n is 6.

5. The vaccine composition of claim 4, wherein the GnRH comprises an amino acid sequence of SEQ ID NO: 2.

6. The vaccine composition of claim 1, wherein when n is 2 or higher, the GnRHs are linked to each other via an alanine-glycine-alanine linker.

7. The vaccine composition of claim 1, wherein the recombinant protein is further fused with a porcine immunoglobulin Fc fragment (pFc2).

8. The vaccine composition of claim 7, wherein the pFc2 comprises an amino acid sequence of SEQ ID NO: 6.

9. The vaccine composition of claim 8, wherein the CTB is fused in the N-terminal direction of GnRH and the pFc2 is fused in the C-terminal direction of GnRH

10. The vaccine composition of claim 1, wherein the recombinant protein is further fused with a chaperone binding protein (BiP).

11. The vaccine composition of claim 10, wherein the BiP comprises an amino acid sequence of SEQ ID NO: 8.

12. The vaccine composition of claim 10, wherein the CTB is fused in the N-terminal direction of GnRH and the BiP is fused in the N-terminal direction of CTB.

13. The vaccine composition of claim 1, wherein the recombinant protein is further fused with His-Asp-Glu-Leu (HDEL).

14. The vaccine composition of claim 13, wherein the HDEL is encoded by a base sequence of SEQ ID NO: 11.

15. The vaccine composition of claim 13, wherein the CTB is fused in the N-terminal direction of GnRH and the HDEL is fused in the C-terminal direction of GnRH.

16. The vaccine composition of claim 1, wherein the recombinant protein is a recombinant protein in which BiP, CTB, GnRH, pFc2, and HDEL are sequentially fused.

17. The vaccine composition of claim 16, wherein the recombinant protein comprises an amino acid sequence of SEQ ID NO: 12.

18. The vaccine composition of any one of claims 1 to 17, wherein the vaccine composition further comprises an adjuvant.

19. The vaccine composition of claim 18, wherein the adjuvant is an Emulsigen-based adjuvant.

20. A method for removing boar taint, the method comprising administering the vaccine composition of any one of claims 1 to 17 to an animal except for a human.

21. The method of claim 20, wherein the animal is a dog, a whale, a cat, a gorilla, a goose, a guinea pig, a pheasant, a camel, a roe deer, a mule, a chicken, a donkey, a dolphin, a pig, a llama, a horse, a water buffalo, a deer, a cow, a reindeer, an alpaca, a yak, a sheep, a goat, an orangutan, a duck, a monkey, a ferret, a rat, a turkey, an ostrich, or a rabbit.

22. A recombinant vector for removing boar taint, the recombinant vector comprising a GnRH gene comprising a base sequence of SEQ ID NO: 3 and a CTB gene comprising a base sequence of SEQ ID NO: 5.

23. The recombinant vector of claim 22, further comprising a polynucleotide encoding pFc2 comprising an amino acid sequence of SEQ ID NO: 6.

24. The recombinant vector of claim 22 or 23, further comprising a polynucleotide encoding BiP comprising an amino acid sequence of SEQ ID NO: 8.

25. The recombinant vector of claim 24, further comprising an HDEL gene comprising a base sequence of SEQ ID NO: 11.

26. The recombinant vector of claim 25, wherein the recombinant vector comprises a polynucleotide encoding an amino acid sequence of SEQ ID NO: 12 or a base sequence of SEQ ID NO: 13.

27. A recombinant protein for removing boar taint produced using the recombinant vector of claim 22.

28. A transformant transformed with the recombinant vector of claim 22.

29. A use of a recombinant protein in which a cholera toxin B subunit (CTB) and n gonadotropin-releasing hormones (GnRH) are fused, wherein n is an integer from 1 to 20 for removing boar taint.

30. A use of a recombinant protein in which a cholera toxin B subunit (CTB) and n gonadotropin-releasing hormones (GnRH) are fused, wherein n is an integer from 1 to 20, for producing a vaccine used to remove boar taint.
